(19) **Europäisches Patentamt / European Patent Office / Office européen des brevets**

(11) **EP 2 191 737 A1**

(12) **EUROPEAN PATENT APPLICATION**
published in accordance with Art. 153(4) EPC

(43) Date of publication:
**02.06.2010 Bulletin 2010/22**

(51) Int Cl.:
*A41D 13/12* (2006.01)    *A61B 5/0205* (2006.01)

(21) Application number: **08831160.0**

(86) International application number:
**PCT/ES2008/070172**

(22) Date of filing: **11.09.2008**

(87) International publication number:
**WO 2009/034218 (19.03.2009 Gazette 2009/12)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **13.09.2007   ES 200702438**

(71) Applicant: **Tag Innovación, S.a.
08290 Cerdanyola Del Vallès (Barcelona) (ES)**

(72) Inventors:
• **JIMENEZ MAROTO, Antonio Manuel
E-08290 Cerdanyola Del Vallès (Barcelona) (ES)**
• **AUBOUY, Laurent
E-08290 Cerdanyola Del Vallès (Barcelona) (ES)**
• **JIMENEZ ROMERO, Javier
E-08290 Cerdanyola Del Vallès (Barcelona) (ES)**
• **SEAZ ZAMORA, José
E-08290 Cerdanyola Del Vallès (Barcelona) (ES)**

(74) Representative: **Pons Ariño, Angel
Glorieta Ruben Dario 4
28010 Madrid (ES)**

(54) **INTELLIGENT WEARING APPAREL**

(57)    Intelligent wearing apparel (1) preferably applicable to the upper part of the body of a user, **characterised in that** it comprises at least one temperature and humidity sensor (2, 3) that measures the temperature and humidity of the layer of air between the body of the user and the inner surface of the apparel (1), an alarm device (13) that produces a sensitive alarm for the user, at least two textile electrodes (11) that measure the user's cardiac rhythm and an electronic plate (4), connected to the temperature and humidity sensor (2, 3), to the alarm device (13) and to the textile electrodes (11), that controls operation thereof, calculating the thermal stress index of the air layer between the user and the apparel, and the thermal stress state of the user, and actuating the alarm device (13) in the event of danger; and wherein a membrane (8) permeable to the air and to the water vapour but impermeable to the liquid water protects the temperature and humidity sensor.

FIG. 1

EP 2 191 737 A1

**Description**

## OBJECT OF THE INVENTION

The main object of the present invention is intelligent wearing apparel comprising a set of sensors and an electronic plate capable of calculating the thermal stress index of the layer of air between the user and the apparel, in addition to the thermal stress state of the user by analysing his/her cardiac rhythm, temperature and relative humidity. Another object of the present invention is wearing apparel that also has an alarm system that alerts the user in advance of the physiological dangers associated to the thermal stress state.

## BACKGROUND OF THE INVENTION

Several documents relating to thermal stress index measurement are known to exist. US 2002009119 discloses a portable device capable of calculating the thermal stress index using several sensors, which include wind speed measurement and other phenomena that can influence it.

US 4611113 discloses a thermal stress index calculator that includes additional sensors, such as a radiation measurement.

Documents that describe sensors integrated into wearing apparel are also known to exist. For example, PCT patent application WO2004107962 discloses a system capable of detecting several factors relating to the apparel user, including his/her cardiac rhythm and temperature, to analyse physiological factors. PCT patent application WO 2006009830 discloses a device capable of determining the user's cardiac rhythm, in addition to his/her body temperature and thermal stress state, using its own calculation system.

## DESCRIPTION OF THE INVENTION

In the prior art, no system integrated into apparel capable of measuring the thermal stress index is disclosed. Neither is any document known to indicate how to determine the risk and/or relative thermal stress state based on environmental temperature and relative humidity conditions of the space between the body of the user and the apparel.

In order to better understand the present document, we will define thermal stress index as a characteristic associated with air, in this case the air contained between the apparel and the skin of the user of said apparel. On the other hand, thermal stress state is a characteristic associated with the person wearing the apparel.

In accordance with an aspect of the present invention, intelligent wearing apparel is described, preferably applicable to the upper part of the body of a user, which comprises

at least one temperature and humidity sensor that measures the temperature and humidity of the layer of air between the body of the user and the inner surface of the apparel,

at least one alarm device that generates a sensitive alarm for the user,

at least two textile electrodes connected to the electronic plate that measure the cardiac rhythm of the user,

an electronic plate connected to the temperature/humidity sensor, to the alarm device and to the textile electrodes, that controls operation thereof, calculating the thermal stress index of the air layer between the user and the apparel, the thermal stress state of the user and activating the alarm device,

and a membrane permeable to the air and the water vapour but impermeable to the liquid water that protects the temperature and humidity sensor.

Therefore, the temperature and humidity sensor or sensors are isolated from the liquid sweat or liquid water on the surface of the user's skin by means of said membrane, which allows the passage of water vapour and air but not liquid water, which prevents the sensor from getting wet and giving false values. This allows measurement of the real thermal stress conditions perceived by the user. In accordance with a preferred embodiment of the invention, the membrane is a micro-perforated textile membrane made of a hydrophobic material.

Additionally and in accordance with another preferred embodiment of the invention, the temperature/humidity sensor or sensors may be integrated onto the electronic plate. In this case, the sensor also has a water-repelling and vapour-breathable membrane but will also have an IP67 shell to effectively protect it from the rest of the electronics. The watertightness of the electronic plate is ensured by a conventional epoxy encapsulation. The plate and sensors will be disposed on the inner part of the apparel, protected by the membrane and facing the user's body.

An alarm device integrated in the fabric of the apparel and also connected to the electronic plate forms the alarm system. This alarm device will be resistant to washing of the apparel and, in accordance with another preferred embodiment of the invention, may be of the sonorous, luminous or vibratory type.

There will normally be two or four textile electrodes, composed of an electrically conductive and adequately disposed material. In accordance with another particular embodiment of the present invention, the intelligent wearing apparel will be intended for the upper part of the body of a user. In this case, the electrodes will be disposed on the inner part of the cuff, ensuring the ergonomic comfort of the apparel and avoiding interference problems with other equipment that may be integrated in the users' clothing.

Additionally, in accordance with another preferred embodiment of the invention, the electronic plate is detachable, in such a manner that the apparel can be separated for washing thereof without risk of damaging the device.

In accordance with a second aspect of the invention, a description is provided of the operation of the previously described intelligent wear apparel, which comprises the following steps:

periodically measure, by means of the, at least, one temperature and humidity sensor, the temperature and humidity of the layer of air between the body of the user and the inner surface of the apparel;

calculate, by means of the electronic plate, the thermal stress index of the air layer between the user and said intelligent wearing apparel based on said temperature and humidity measurements;

in the event that the thermal stress index exceeds a critical thermal stress index, measure the user's cardiac rhythm by means of the, at least, two textile electrodes; and

in the event that the user's cardiac rhythm exceeds a critical cardiac rhythm, produce an alarm by means of the, at least, one alarm device.

Calculation of the thermal stress index can be carried out in accordance with any of the known formulas in the state of the art, and the limits as of which the user's health is at risk are also known. The electronic plate will calculate the thermal stress index and will start measuring the user's cardiac rhythm when it exceeds an established limit as of which the user's health is at risk. If the cardiac rhythm exceeds a critical value that can pose a risk to the user, the alarm device will produce a sensitive alarm for the user, indicating that his/her cardiac rhythm has exceeded the risk threshold. The critical value of the cardiac rhythm will depend on the person's age and there are known ways of calculating it.

Additionally, in accordance with another preferred embodiment of the invention, the interval between temperature and humidity measurements decreases as the thermal stress index rises. This is aimed at saving on energy consumption, in such a manner that the duration of the electronic device will exceed that of the useful life of the apparel, avoiding the need for maintenance or spare parts. Therefore, when the level of thermal stress is low, the intervals between measurements are greater and it is not necessary to measure cardiac rhythm. However, as thermal stress increases, and therefore the risk posed to the user's health, the measurement frequency also increases. On reaching a critical thermal stress value it starts measuring cardiac rhythm and, when said rhythm exceeds a determined value, it generates an alarm.

## DESCRIPTION OF THE DRAWINGS

In order to complement this description and with the object of helping to better understand the characteristics of the invention, a set of drawings in accordance with a preferred example of practical embodiment thereof has been included as an integral part of said description, wherein the following have been represented in an illustrative and non-limiting manner:

Figure 1 shows a general view of a particular embodiment of the apparel in accordance with the present invention.

Figure 2 shows a schematic view of a temperature and humidity sensor disposed in the apparel in accordance with an embodiment of the present invention.

Figure 3 shows a schematic view of a temperature/humidity sensor integrated with an electronic plate, disposed on apparel in accordance with a particular embodiment of the apparel in accordance with the present invention.

## PREFERRED EMBODIMENT OF THE INVENTION

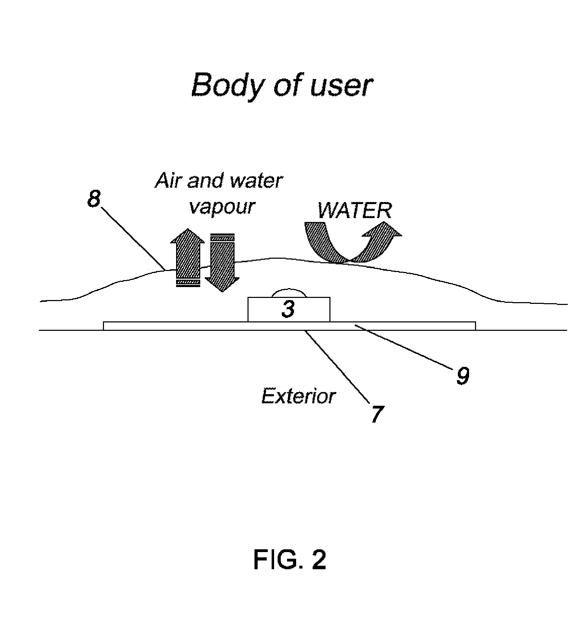

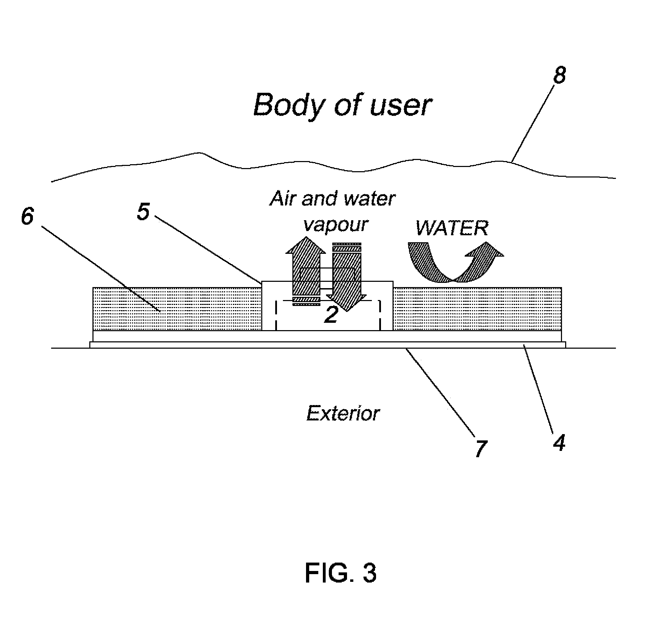

In accordance with a preferred embodiment of the invention, the wearing apparel is a shirt or polo shirt (1) wherein two temperature and humidity sensors (2, 3) are disposed. A temperature and humidity sensor (2) is integrated in the electronic plate (4) that controls the operation of the rest of the devices and is installed close to the neck of the polo shirt (1). This temperature and humidity sensor (2) is represented in Fig. 3, where we can observe how said temperature and humidity sensor (2) is fixed to the electronic plate (4) and is protected by an IP67-type encapsulation (5). A layer of epoxy resin (6) protects the rest of the electronic plate (4), which is in turn fixed to the fabric (7) of the polo shirt. Finally, we can observe the membrane (8) that is permeable to the water vapour and air that separates the unit from the user's skin. The second temperature and humidity sensor (3) represented in Fig. 2 is installed on a support (9) fixed to one side of the fabric (7) of the polo shirt (1) and is connected by a guide wire (10) to the electronic plate. As in the case of the first temperature and humidity sensor (2), a membrane (8) that is permeable to the water vapour and air, but impermeable to the liquid water, separates it from the user's skin. A pair of textile electrodes (11) is integrated into the cuffs of the polo shirt (1), in such a manner that they are permanently in contact with the user's skin and are also connected to the electronic plate (4) by means of a guide wire (12).

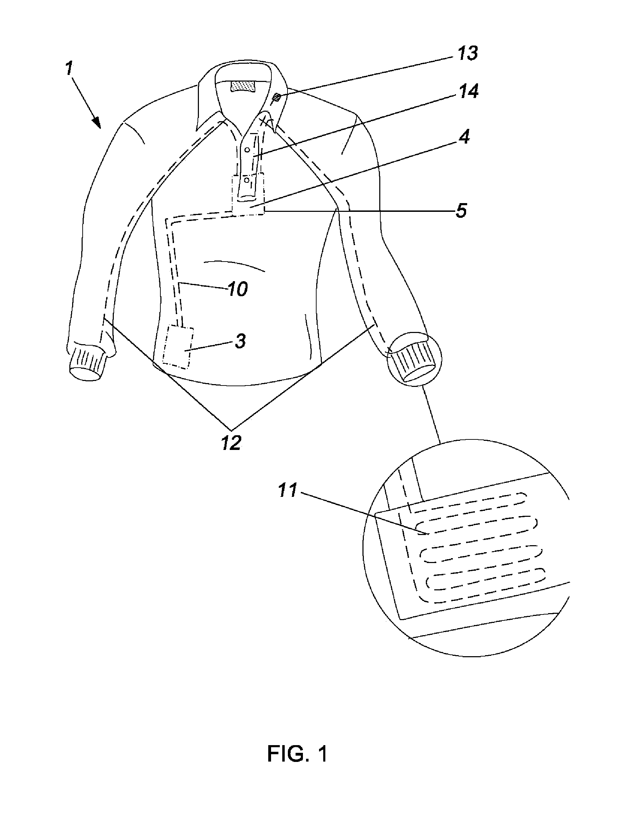

In Fig. 1 we can also observe a piezoelectric hummer (13) installed in the neck of the polo shirt (1) and joined by means of a guide wire (14) to the electronic plate (4).

Although the water resistance of the sensors (2, 3) and the electronic plate (4) is sufficient to support some washes and immersion in water, the fastenings thereof to the polo shirt (1) are detachable, in such a manner that they can be separated therefrom to prevent them from becoming damaged.

Operation of the polo shirt (1) is as follows: the electronic plate (4) sends an order to the temperature and humidity sensors (2, 3) to measure the temperature and humidity with a determined frequency and, for each temperature and humidity value measured, calculates the thermal stress index in accordance with the following known formula:

$$HI = c_1 + c_2T + c_3R + c_4TR + c_5T^2 + c_6R^2 + c_7T^2R + c_8TR^2 + c_9T^2R^2$$

where:

HI = Thermal stress index (in degrees Fahrenheit)
T = Ambient dry-bulb temperature (in degrees Fahrenheit)
R = Relative humidity (as a percentage)
$C_1$ = -42.379
$C_2$ = 2.04901523
$C_3$ = 10.1433127
$C_4$ = -0.22475541
$C_5$ = -6.83783 x $10^{-3}$
$C_6$ = -5.481717 x $10^{-2}$
$C_7$ = 1.22874 x $10^{-3}$
$C_8$ = 8.5282 x $10^{-4}$
$C_9$ = -1.99 x $10^{-6}$

It is known that, if the thermal stress index exceeds a value of 105, certain risks are posed to the user's health, said risk becoming extreme when the thermal stress index exceeds a value of 129. Therefore, in this particular embodiment, a value of 115 is considered to be the critical thermal stress index. When one of the thermal stress index measurements exceeds this critical value, the electronic plate (4) starts measuring the cardiac rhythm through the textile electrodes (11).

It is also known that a person's health is at risk when his/her cardiac rhythm exceeds (220-person's age) beats. In this particular embodiment, a value of 205 beats per minute minus the person's age is considered to be the critical cardiac rhythm, in such a manner that when this value is exceeded the electronic plate (4) starts up the piezoelectric hummer (13), which will alert the user that his/her health is at risk.

Additionally, the frequency with which the temperature and humidity is measured and with which the critical stress index is calculated increases parallel to the thermal stress index value, as the greater the value the greater the risk to the health of the person wearing the apparel.

**Claims**

1. Intelligent wearing apparel (1) preferably applicable to the upper part of the body of a user, **characterised in that** it comprises,

   at least one temperature/humidity sensor (2, 3) that measures the temperature and humidity of the layer of air between the body of the user and the inner surface of the apparel (1);
   at least one alarm device (13) that produces a sensitive alarm for the user;
   at least two textile electrodes (11), connected to the electronic plate (4), that measure the user's cardiac rhythm; and
   an electronic plate (4), connected to the temperature and humidity sensor (2, 3), to the alarm device (13) and to the textile electrodes (11) that controls operation thereof and calculates the thermal stress index of the air layer between the user and the apparel and actuates the alarm device (13);
   and a membrane (8) permeable to the air and water vapour but impermeable to the liquid water that protects the temperature/humidity sensor (2, 3).

**2.** Intelligent wearing apparel (1),in accordance with claim 1, **characterised in that** the membrane (8) permeable to the air is a micro-perforated textile membrane made of a hydrophobic material.

**3.** Intelligent wearing apparel (1), in accordance with claim 1, **characterised in that** the alarm device (13) is selected from among the following list: sonorous, luminous and vibratory.

**4.** Intelligent wearing apparel (1), in accordance with claim 1, **characterised in that** the temperature and humidity sensor (2) is integrated onto the electronic plate (4).

**5.** Intelligent wearing apparel (1), in accordance with claim 1, **characterised in that** the electronic plate (4) is detachable.

**6.** Intelligent wearing apparel (1), in accordance with claim 1, **characterised in that** the textile electrodes (11) are disposed on the cuffs of said apparel (1).

**7.** Operating process of the intelligent wearing apparel (1) described in any of the preceding claims, **characterised in that** it comprises the following steps:

periodically measure, by means of the, at least, one temperature/humidity sensor (2, 3), the temperature and humidity of the layer of air between the body of the user and the inner surface of the apparel (1);
calculate, by means of the electronic plate (4), the thermal stress index of the air layer between the user and said intelligent wear apparel (1) based on said temperature and humidity measurements;
in the event that the thermal stress index exceeds a critical thermal stress index, measure the user's cardiac rhythm by means of the, at least, two textile electrodes; and
in the event that the user's cardiac rhythm exceeds a critical cardiac rhythm, produce an alarm by means of the, at least, one alarm device.

**8.** Process, in accordance with claim 7, **characterised in that** the interval between the temperature and humidity measurements decreases when the thermal stress index increases.

**9.** Process, in accordance with claim 7, **characterised in that** the step of producing an alarm comprises producing an alarm of one or several of the following types: luminous, sonorous and vibratory.

FIG. 1

Body of user

Air and water vapour

WATER

Exterior

3

8

9

7

FIG. 2

Body of user    8

Air and water
vapour    WATER

6    5

2

Exterior

7    4

FIG. 3

# INTERNATIONAL SEARCH REPORT

| International application No. |
|---|
| PCT/ ES 2008/070172 |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)

A41D 13/00, A61B 5/00

Documentation searched other than minimum documentation to the extent that such documents are included in the fields searched

Electronic data base consulted during the international search (name of data base and, where practicable, search terms used)

INVENES,EPODOC, WPI, TXT, NPL, Internet

**C. DOCUMENTS CONSIDERED TO BE  RELEVANT**

| Category* | Citation of document, with indication, where appropriate, of the relevant passages | Relevant to  claim No. |
|---|---|---|
| X | WO 2007050650 A1 (SENSATEX INC ; SHARMA VIKRAM) | 1-3,5 |
| Y | paragraphs [141-205]; images. | 4,6 |
| Y | US 2007197878 A (SHKLARSKI et al.) 23.08.2007, 03.05.2007, paragraphs [30-77]; images. | 4,6 |
| Y | DE 102004030261 A1 (INST TEXTIL & FASERFORSCHUNG) 19.01.2006, the whole document. | 1-6 |
| Y | US 6315009 A (JAYARAMAN et al.) 13.11.2001, the whole document. | 1-6 |
| A | EP 1506738 A1 (TAM TELESANTE) 16.02.2005, the whole document. | 1-9 |
| A | "Recent developments and trends in biomedical sensors"; Mehmet Engin et al.; Measurement 37 (2005) 173–188; Disponible in www.elsevier.com/ locate/measurement desde the 15.12.2004. | 1-6 |

☐ Further documents are listed in the continuation of Box C.          ☒ See patent family annex.

| | |
|---|---|
| *    Special categories of cited documents:<br>"A"   document defining the general state of the art which is not considered to be of particular relevance.<br>"E"   earlier document but published on or after the international filing date<br><br>"L"   document which may throw doubts on priority claim(s) or which is cited to establish the publication date of another citation or other special reason (as specified)<br>"O"   document referring to an oral disclosure use, exhibition, or other means<br>"P"   document published prior to the international filing date but later than the priority date claimed | "T"   later document published after the international filing date or priority date and not in conflict with the application but cited to understand the principle or theory underlying the invention<br><br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other documents , such combination being obvious to a person skilled in the art<br><br>"&"   document member of the same patent family |
| Date of the actual completion of the international search<br><br>27.January.2009          (27.01.2009) | Date of mailing of the international search report<br><br>**(02/02/2009)** |
| Name and mailing address of the ISA/<br>O.E.P.M.<br><br>Paseo de la Castellana, 75 28071 Madrid, España.<br>Facsimile No.   34 91 3495304 | Authorized officer<br><br>P. Tauste Ortiz<br><br>Telephone No. +34 91 349 85 46 |

Form PCT/ISA/210 (second sheet) (July 2008)

## INTERNATIONAL SEARCH REPORT

Information on patent family members

International application No.

PCT/ ES 2008/070172

| Patent document cited in the search report | Publication date | Patent family member(s) | Publication date |
|---|---|---|---|
| WO 2007050650 A | 03.05.2007 | US 2007089800 A | 26.04.2007 |
| US 2007197878 A | 23.08.2007 | WO 2006006159 A | 19.01.2006 |
| WO 2006000345 A | 05.01.2006 | US 2008091097 A | 17.04.2008 |
| | | DE 102004030261 A | 19.01.2006 |
| | | EP 1773195 A | 18.04.2007 |
| | | EP 20050762685 | 17.06.2005 |
| | | CN 1976632 A | 06.06.2007 |
| | | JP 2008503287 T | 07.02.2008 |
| | | | 07.02.2008 |
| | | | 07.02.2008 |
| US 6315009 B | 13.11.2001 | WO 9964657 A | 16.12.1999 |
| | | CA 2295534 A | 16.12.1999 |
| | | AU 6128699 A | 30.12.1999 |
| | | EP 1041927 A | 11.10.2000 |
| | | EP 19990948028 | 12.05.1999 |
| | | US 6145551 A | 14.11.2000 |
| | | CN 1274270 A | 22.11.2000 |
| | | US 6381482 B | 30.04.2002 |
| | | CA 2428919 A | 23.05.2002 |
| | | WO 0240756 A | 23.05.2002 |
| | | WO 0240091 A | 23.05.2002 |
| | | AU 3648102 A | 27.05.2002 |
| | | JP 2002517301 T | 18.06.2002 |
| | | AU 750158 B | 11.07.2002 |
| | | US 6474367 B | 05.11.2002 |
| | | WO 02100200 A | 19.12.2002 |
| | | EP 1339903 A | 03.09.2003 |
| | | EP 20010986012 | 14.11.2001 |
| | | MXPA 03004222 A | 22.09.2003 |
| | | US 6687523 B | 03.02.2004 |
| | | CN 1486376 A | 31.03.2004 |
| | | JP 2004514068 T | 13.05.2004 |
| | | US 6970731 B | 29.11.2005 |
| EP 1506738 A | 16.02.2005 | EP 20040291994 | 04.08.2004 |
| | | US 2005034485 A | 17.02.2005 |
| | | US 7319895 B | 15.01.2008 |
| | | FR 2858758 AB | 18.02.2005 |

Form PCT/ISA/210 (patent family annex) (July 2008)

## INTERNATIONAL SEARCH REPORT

International application No.

PCT/ ES 2008/070172

CLASSIFICATION OF SUBJECT MATTER

*A41D 13/12* (2006.01)
*A61B 5/0205* (2006.01)

Form PCT/ISA/210 (extra sheeet) (July 2008)

**EP 2 191 737 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2002009119 A
- US 4611113 A
- WO 2004107962 PCT
- WO 2006009830 PCT